# EUROPEAN PATENT APPLICATION

(11) **EP 0 562 185 A1**
(43) Date of publication of application: **29.09.1993**
(21) Application number: 92302275.0
(22) Date of filing: 17.03.1992
(51) Int. Cl.: C07D 207/08, C07D 405/06, A61K 31/40

(54) **3-(Arylmethyl)-1-(3'-diethylaminopropyl) pyrrolidines and process of preparation thereof**

(71) Applicant: Council of Scientific and Industrial Research, New Delhi 110 001 (IN)
(72) Inventor: De, Dibyendu, Lucknow 226001 (IN); Seth, Manju, Lucknow 226001 (IN); Puri, Sunil Kumar, Lucknow 226001 (IN); Chandra, Subhash, Lucknow 226001 (IN); Bhaduri, Amiya Prasad, Lucknow 226001 (IN)
(74) Representative: Hardisty, David Robert

(57) **Abstract**

The novel compounds 3-(arylmethyl)-1-(3'-diethylaminopropyl) pyrrolidines are disclosed which have the general formula: 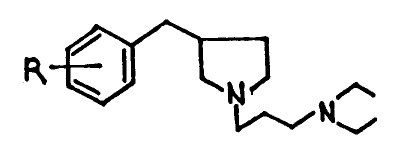
wherein R represents an alkoxy group having 1 to 6 carbon atoms, like methoxy, ethoxy, propoxy or butoxy and a process for preparing the same. Particularly, the invention provides the compound having the above said formula wherein the alkoxy substituent is in the 4 position of the benzene ring or the substituent methylenedioxy at 3 and 4 position. The compounds have the property of reversing the resistance of chloroquine resistant strains of the malarial parasite P. berghi in vivo and P. falciparum in vitro and can be used for reversing the resistance of human malarial parasites, particularly P. falciparum. The invention also includes compositions containing the new compounds and an anti-malarial drug, preferably chloroquine.

## Description

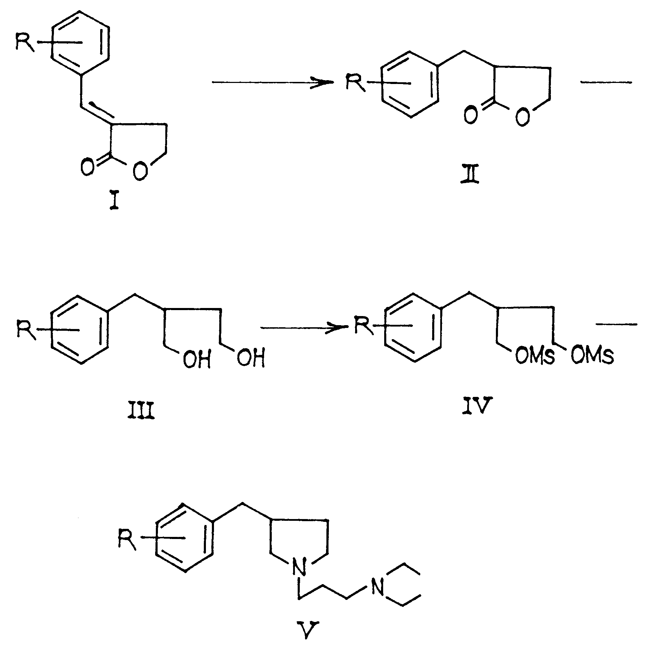

The present invention relates to novel compounds, namely 3-(arylmethyl)-1-(3'-diethylaminopropyl) pyrrolidines as shown in formula V of the drawing accompanying the specification. The invention relates also to a process for the preparation of 3-(arylmethyl)-1-(3'-diethylaminopropyl) pyrrolidines which have the formula V shown of the drawings accompanying the specification, where R represents an alkoxy group like methoxy, dimethoxy, methylenedioxy. These compounds have the property of reversing the resistance of chloroguine-resistant strains of malarial parasites. Oral administration of these new compounds along with an antimalarial drug, such as chloroquine, would be useful for the treatment of falciparum malaria affected patients who do not respond to chloroquine and the other antimalarial drugs.

Chloroquine is a useful and widely used drug for the treatment of malaria caused by the malarial parasite, Plasmodium falciparum. However, there are strains of P. falciparum which do not respond to chloroquine. A new approach to treatment of such cases of malaria is to administer the compounds which will reverse the chloroquine resistance of the parasite and make the latter susceptible to the action of the drug chloroquine.

Till now certain calcium channel blockers of the type of verapamil (α-isopropyl-3,4-dimethoxy-α-[[[2-N-(3,4-dimethoxyphenyl)ethyl]-N-methylamino]ethyl] phenylacetonitrile), Nifedipine and analogs of these compounds have been reported in literature. Reference in this connection is made in the articles of 1. Krogstad, D.J., Gulzman, I.Y., Kyle, D.E., Oduola, A.M.J., Martin, S.K., Milhous, W.K. and Schlesinger, P.H. (1987) Science, 238, 1283-1285; 2. Milhous, W.K., Kyle, D.E., Oduola, A.M.J., Martin, S.K., Boudrean, B. Schuster B.G. and Davidson, D.E. Calcium Channel Blockers and Malaria, 3rd International Congress on Malaria and Babesiosis, Annecy-France, September 8, 1987 and 3. Martin, S.K., Oduola, A.M.J. and Milhous, W.K. (1987) Science 235, 899-901 to have the property of reversing the chloroquine resistance of P. falciparum in Aotus monkey.

The particular dose at which verapamil can reverse chloroquine resistance of P. falciparum parasite in vivo was found to be toxic to Aotus monkey and hence verapamil cannot be used clinically. Nifedipine [3,5-dicarbomethoxy-1,4-dihydro-2,6-dimethyl-4-(2-nitrophenyl)pyridine] at low dose in combination with chloroquine showed suppression of parasitaemia in Aotus monkey. Retreatment with higher doses of nifedipine plus chloroquine cured the infection but nifedipine too showed significant toxicity in Aotus monkey.

Compared to the compounds of the prior art, the compounds as per the present invention have the following distinguishing features:
(i) they have the property of reversing the chloroquine resistance of resistant strains of malarial parasites, P. berghi in vivo and P. falciparum in vitro and can be used for reversing the resistance of human malarial parasites, particularly P. falciparum.
(ii) they thus make the parasites susceptible to the action of the drug chloroquine.

Accordingly the present invention provides in one aspect a process for the preparation of novel compounds 3-(arylmethyl)-1-(3'-diethylaminopropyl) pyrrolidines of the formula V shown in the drawing accompanying the specification where R respresents an alkoxy group like methoxy, dimethoxy, methylene dioxy which comprises:
(i) hydrogenating α-(arylmethylene)-γ-butyrolactone of the formula II,
(ii) hydride reduction by conventional methods of the resulting compound of the formula II to get 2-(arylmethyl)1,4-butanediol of the formula III,
(iii) mesylating 2-(arylmethyl)-1,4-butanediol of the formula III by known methods to give 2-(arylmethyl)-1,4-dimesyloxy-butane of the formula IV and
(iv) reacting 2-(arylmethyl)-1,4-dimesyloxybutane of the formula IV with diethylaminopropylamine to give the compound of the formula V where R has the meaning given above.

The most active compound falling within the formula V is 3-(4-methoxybenzyl)-1-(3'-diethylaminopropyl)pyrrolidine. This compound was tested for reversal of chloroquine-resistance of P. berghi infection in mice at two doses. In the first test chloroquine (50 mg/kg) along with the compound (4 to 16 mg/kg) was administered on day one, and was followed by the tested compound only (4 to 16 mg/kg) for three more consecutive days. In the second test, chloroquine (50 mg/kg) and the test compounds were administered together for four consecutive days. Cent percent reversal of chloroquine resistance was observed in both the tests.

The present invention particularly provides the 3-(arylmethyl)-1-(3'diethylaminopropyl)pyrrolidines of the formula V having as substituents on the phenyl ring (ring A), an alkoxy group having 1 to 6 carbon atoms, preferably methoxy, ethoxy, propoxy or butoxy, at position 4, or a methylene-dioxy group at positions 3 and 4 and a process for preparing the said compounds.

The hydrogenation steps may preferably be effected using 10% Pd-C in the presence of solvents like tetrahydrofuran, methanol, ethanol, isopropanol, cyclohexanol, t-butanol, dioxane etc. The reduction of the α-(arylmethyl)-γ-butyrolactone may be affected using sodium borohydride, potassium borohydride, sodium cyanoborohydride or diborane in the presence of solvents like methanol, isopropanol, tert-butanol at a temperature in the range of 60-100^{o}C.

The mesylation may be effected by using mesyl chloride, p-toluenesulfonyl chloride or trimethylsilyl chloride in the presence of tertiary amines like triethylamine, tributylamine, tripropylamine and solvents like benzene, ether, tetrahydrofuran, dichloromethane at a temperature in the range of -10°C to 25°C. The last step may be effected by using tertiary amine, like triethylamine, tributylamine, tripropylamine, and solvent, such as benzene, ether, dichloromethane, at a temperature in the range of 40 to 80°C.

The invention is illustrated by the examples given below which should not be construed to limit the scope of the invention.

### Example 1:

### Preparation of 3-(4-methoxybenzyl)-1-(3'-diethylamino-propyl) pyrrolidine

### Step (i) Preparation of α-(4-methoxybenzyl)-γ-butyrolactone (II)

To a solution of α-(4-methoxybenzylidene)-γ-butyrolactone of the formula (I) (9.1 g) in ethylacetate (250 ml) at room temperature (35^{o}C) was added 10% Pd-C (2.0 g). The reaction mixture was hydrogenated (at pressure 3.5 kg-cm⁻²) for 24 hrs at room temperature and then filtered. The Pd-C was washed 2-3 times with a little ethylacetate. The ethylacetate was evaporated off from combined filtrate in vacuo and the residue was washed 3-4 times with hexane-benzene (9:1) mixture to give α-(4-methoxybenzyl)-γ-butyrolactone of the formula (II) as glass, yield 8.5 g.

### Step (ii) Preparation of 2-(4-methoxybenzyl)-1,4-butanediol (III)

To an ice-cooled solution of α-(4-methoxy benzyl)-γ-butyrolactone of the formula (II) (8.5 g) in methanol (60 ml) was added sodium borohydride (6 g) portionwise under constant stirring which was continued for another 1 hr. keeping the reaction mixture at 0-5^{o}C. Then the mixture was refluxed for 6-7 hrs. Methanol was evaporated off in vacuo. Water (80 ml) was added to the residue which was then extracted with ethyl acetate (3 x 75 ml). The ethyl acetate extract was washed with water (2 x 60 ml), dried over anhydrous sodium sulphate and the solvent was removed in vacuo. The residue of 2-(4-methoxy-benzyl)-1,4-butanediol of the formula (III) thus obtained was washed with hexane (2 x 40 ml) to give an oil yield 6.8 g.

### Step (iii) Preparation of the dimesylate of 2-(4-methoxybenzyl)-1,4-butanediol (IV)

In a three-necked round bottom flask containing an ice-cooled solution of 2-(4-methoxybenzyl)-1,4-butanediol of the formula (III) (6.8 g) in dry benzene (120 ml) were added mesyl chloride (5.4 ml) and triethylamine (11.0 ml) dropwise under constant stirring. The mixture was kept at 0-5^{o}C for 3-4 hrs. with constant stirring. Water (100 ml) was added to the reaction mixture which was then extracted with ethyl acetate (3 x 60 ml). The ethyl acetate extract was washed with brine (50%, 70 ml) and then with water (2 x 50 ml) and dried over anhydrous sodium sulphate. The solvent was removed in vacuo to give residue of dimesylate of 2-(4-methoxybenzyl)-1,4-butanediol of the formula (IV) as an oil yield 11 g.

### Step (iv) Preparation of 3-(4-methoxybenzyl)-1-(3'-diethylaminopropyl)-pyrrolidine (V)

To a solution of the dimesylate of 2-(4-methoxybenzyl)-1,4-butanediol of the formula (IV) (11.0 g) in dry benzene (100 ml) was added a solution of N,N-diethylaminopropylamine (16 ml) and triethylamine (12.4 ml) in dry benzene (25 ml) dropwise under constant stirring. The reaction mixture was stirred at room temperature for 1 hr, then refluxed for 5-6 hrs. and then cooled to room temperature. Water (50 ml) was added and it was extracted with ethyl acetate (3 x 80 ml). The ethyl acetate extract was washed with water (2 x 70 ml), dried over anhydrous sodium sulphate and the solvent was evaporated in vacuo. The oily residue thus obtained was chromatographed over basic alumina using benzene-chloroform (4:1) as eluant. Evaporation of solvent from the eluate gave 3-(4-methoxybenzyl)-1-(3'-diethylaminopropyl)-pyrrolidine of the formula (V) as an oil yield 7.3 g.

### Example 2:

### Preparation of 3-(3,4-methylenedioxybenzyl)-1-(3'-diethylaminopropyl)-pyrrolidine (V)

### Step (i) Preparation of α-(3,4-methylenedioxybenzyl)-γ-butyrolactone (II)

To a solution of α-(3,4-methylenedioxybenzylidene)-γ-butyrolactone of the formula (I) (9.85 g) in ethyl acetate (250 ml) at room temperature was added 10% Pd-C (2.0 g). The reaction mixture was hydrogenated (at pressure 3.5 kg-cm⁻²) at room temperature for 24 hrs. and then filtered. The Pd-C was washed 2-3 times with a little ethyl acetate. The combined filtrate was concentrated to about 10ml. The solid α-(3,4-methylenedioxybenzyl)-γ-butyrolactone of the formula (II) which separated out was filtered and recrystallised from chloroform-hexane, m.p.66-67^{o}C, yield 9.45 g.

### Step (ii) Preparation of 2-(3,4-methylenedioxybenzyl)-1,4-butanediol (III)

To an ice cooled solution of α-(3,4-methylenedioxybenzyl)-γ-butyrolactone of the formula II (9.45 g) in methanol (60 ml) was added sodium borohydride (6 g) portionwise under constant stirring, which was continued for 1 hr keeping reaction mixture at 0-5^{o}C. Then the reaction mixture was refluxed for 8-9 hrs. Methanol was evaporated off in vacuo, water (80 ml) was added to the residue which was extracted with water (2 x 60 ml), dried over anhydrous sodium sulphate and the solvent removed in vacuo. The oily residue thus obtained was washed with hexane (2 x 50 ml) to give 2-(3,4-methylenedioxybenzyl)-1,4-butanediol of the formula (III) as an oil yield 8.2 g.

### Step (iii) Preparation of dimesylate of 2-(3,4-methylenedioxybenzyl)-1,4-butanediol (IV)

In a three-necked round bottom flask containing an ice-cooled solution of 2-(3,4-methylenedioxybenzyl)-1,4-butanediol of the formula (III) (8.2 g) in dry benzene (130 ml) were added mesyl chloride (5.4 ml) and triethylamine (11.0 ml) dropwise under constant stirring. The reaction mixture was stirred at 0-5^{o}C for 3-5 hrs. then water (100 ml) was added to it, and it was extracted with ethyl acetate (3 x 70 ml). The ethyl acetate extract was washed with brine (50%, 80 ml) and then with water (2 x 60 ml), dried over anhydrous sodium sulphate. The solvent was evaporated off in vacuo to give the dimesylate of 2-(3,4-methylenedioxybenzyl)-1,4-butanediol of the formula (IV) as an oil yield 12.57 g.

### Step (iv) Preparation of 3-(3,4-methylenedioxybenzyl)-1-(3'-diethylaminopropyl)-pyrrolidine (V)

To a solution of the dimesylate of 2-(3,4-methylenedioxybenzyl)-1,4-butanediol of the formula (12.57 g) in dry benzene (100 ml) was added a solution of N,N-diethylaminopropylamine (16 ml) and triethylamine (12.4 ml) in dry benzene (25 ml) dropwise under constant stirring. The reaction mixture was stirred at room temperature for 1 hr, then refluxed for 5-6 hrs. and then cooled at room temperature. Water (60 ml) was added and the mixture was extracted with ethyl acetate (3 x 85 ml). The ethyl acetate extract was washed with water (2 x 75 ml), dried over anhydrous sodium sulphate and the solvent removed in vacuo. The oily residue thus obtained was chromatographed over basic alumina using benzene-chloroform (4:1) as eluant. Evaporation of solvent from the eluate gave pure 3-(3,4-methylenedioxybenzyl)-1-(3'-diethylaminopropyl)pyrrolidine of the formula (V) as an oil yield 7.8 g.

It is to be understood in the compounds of this invention according to formula V, there is usually only one R group, but also there may be two R groups, which may be the same or different. It is preferred that when R is methylenedioxy there are two such groups in the ring.

## Claims

1. A compound 3-(arylmethyl)-1-(3'-diethylaminopropyl) of the formula V 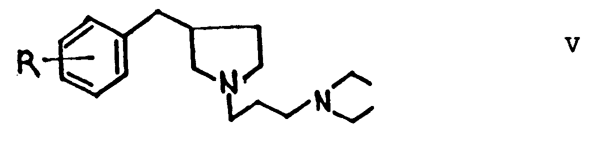 where R represents an alkoxy group having 1 to 6 carbon atoms, preferably methoxy, ethoxy, propoxy or butoxy, or the methylenedioxy group.

2. A compound as claimed in claim 1 wherein the substituent R is in 4 position.

3. A compound as claimed in claim 1 wherein the substituent R is methylenedioxy at both the 3 and 4 positions.

4. 3-(4-methoxybenzyl)-1-(3'-diethylaminopropyl) pyrrolidine.

5. A process for the preparation of 3-(arylmethyl)-1-(3'-diethylaminopropyl) as claimed in claim 1, where R represents an alkoxy group which process comprises:
(i) hydrogenating an appropriate substituted α-(arylmethylene)-γ-butyro-lactones of the formula I of the accompanying drawings to give the corresponding α-(arylmethyl)-γ-butyrolactones of the formula II,
(ii) reducing the compound of the formula II to give the corresponding 2-(arylmethyl)-1,4-butanediol of the formula III,
(iii) mesylating the compound of the formula III to give the corresponding protected 2-(arylmethyl)-1,4-butanediol of the formula IV, and
(iv) reacting the compound of the formula IV with diethylaminopropylamine to give a compound as claimed in claim 1.

6. A process as claimed in claim 5, wherein the hydrogenation (step 1) is effected using 10% Pd-C catalyst in the presence of a solvent, preferably methanol, ethanol, isopropanol, cyclohexanol, dioxane or tetrahydrofuran.

7. A process as claimed in either of claims 5 or 6, wherein the hydrogenation is carried out at a temperature between 20^{o}C and 60^{o}C.

8. A process as claimed in any one of claims 5 to 7, wherein the reduction (step ii) is a hydride reduction effected using sodium borohydride or potassium borohydride preferably at a temperature of 60^{o}C to 100^{o}C.

9. A process as claimed in any one of claims 5 to 8, wherein the mesylating step is effected by reaction with a hydroxy group protecting agent, preferably mesyl chloride, p-toluene sulfonyl chloride or trimethyl silyl chloride.

10. A process as claimed in claim 9, wherein the mesylation is effected in the presence of a tertiary amine selected from triethylamine, tripropylamine or tributylamine.

11. A process as claimed in either of claims 9 or 10, wherein the mesylation is carried out at a temperature of 10^{o}C to 25^{o}C.

12. A process as claimed in any one of claims 5 to 11, wherein the dimesyl derivatives of 2-(arylmethyl)-1,4-butanediol of the formula IV is reacted with diethylamino propylamine in the presence of a tertiary amine, preferably triethylamine, tripropyl amine or tributylamine.

13. A process as claimed in claim 12, wherein the reaction is carried out at a temperature of 40^{o}C to 80^{o}C.

14. A pharmaceutical composition comprising a compound as claimed in any one of claims 1 to 4 and an anti-malarial drug, preferably chloroquine.

15. The use of a compound as claimed in any one of claims 1 to 4 in the preparation of a pharmaceutical composition, especially a composition for the treatment of malaria.
